# EUROPEAN PATENT SPECIFICATION

(11) **EP 2 448 544 B1**
(45) Date of publication and mention of the grant of the patent: **21.08.2013**
(21) Application number: 10744541.3
(22) Date of filing: 01.07.2010
(51) Int. Cl.: A61K 8/11, A61K 8/25, A61K 8/58, A61K 8/891, A61K 8/895, A61Q 1/02

(54) **COSMETIC COMPOSITION COMPRISING ENCAPSULATED SILICONE COMPOUNDS**
KOSMETISCHE ZUSAMMENSETZUNGEN MIT GEKAPSELTEN SILIKONVERBINDUNGEN
COMPOSITION COSMÉTIQUE COMPRENANT DES COMPOSÉS DE SILICONE ENCAPSULÉS

(30) Priority: 01.07.2009 US 222223 P; 01.07.2009 FR 0954488
(43) Date of publication of application: 09.05.2012
(73) Proprietor: L'Oréal, 75008 Paris (FR)
(72) Inventor: SIMONNET, Jean-Thierry, F-94230 Cachan (FR); GAVILLON, Roxane, F-91170 Viry Chatillon (FR)
(74) Representative: Le Blainvaux Bellegarde, Françoise
(86) International application number: PCT/EP2010/059345
(87) International publication number: WO 2011/000902

(56) References cited:
- EP-A1- 1 941 930
- WO-A2-2008/087325

## Description

The present invention relates to a cosmetic composition comprising at least one organopolysiloxane compound X having at least two alkenyl unsaturated groups, at least one organohydrogensiloxane compound Y and at least one hydrosilylation catalyst, at least one of the compounds X or Y being encapsulated in silica shell microcapsules.

The compositions considered according to the invention are more particularly intended for caring for and/or making up keratin materials and especially the skin, lips and integuments.

Generally, cosmetic compositions are intended to provide an aesthetic effect and this aesthetic effect is generally obtained by the formation of a film of make-up and/or care product on the support in question such as, for example, the face, lips, eyelashes, nails and hair.

For obvious reasons, optimization of the comfort, staying power and/or transfer-resistance qualities of these films is a constant concern in the cosmetic field.

It is known that certain systems comprising silicone compounds prove to be capable, by simple contacting of these compounds, where appropriate in the presence of a catalyst, of producing silicone polymer films. Thus, silicone compounds referred to as compound X and compound Y, as defined below, prove capable of polymerizing in situ, at atmospheric pressure and room temperature and of forming films that are advantageously biocompatible, non-tacky, slightly opalescent or even peelable. Such systems are especially described in documents WO 01/96 450 and GB 2 407 496. However, given the high reactivity of these compounds, it is imperative to package them separately in order to prevent the premature formation of a film.

Consequently, the use of these systems in the care and/or make-up field imposes a mode of packaging or even of application similar to that of a double action in order to guarantee that the mixing of the two silicone compounds forming the system, carried out where appropriate in the presence of a catalyst, only takes place in contact with the support in question or is only performed extemporaneously just before its application to the support.

It is clear that this need to package the two silicone compounds separately, or where appropriate the two silicone compounds and the catalyst separately, represents a constraint for both the formulator and the user, which it would be desirable to dispense with.

It is thus known from application EP-A-1 935 454 how to formulate such a system as a single composition and encapsulating at least one of the components of the system in polymer shell microcapsules. However, certain polymers such as polycaprolactone, polylactides, polyglucolides, polymers of 3-hydroxybutyric acid, vinyl chloride/vinyl acetate copolymers, methacrylic acid/methyl methacrylate copolymers, polyalkylene adipates and polyester polyols prove less advantageous during storage of the composition at temperatures above 40°C: indeed, at these high temperatures, and especially during storage over 2 months at 45°C, the polymer capsules partly lose their sealing ability and a portion of the components of the system escapes from the capsules and can therefore react when they come into contact at the very core of the composition. The composition then no longer exhibits good storage stability properties and under these conditions the system of silicone components begins to react by premature crosslinking within the composition before its use and its application to the keratin materials. Such a composition does not exhibit its best ability to form a film during the contacting of the components during the rupture of the capsules since the reaction is already initiated within the composition.

The objective of the present invention is therefore to improve the storage stability of capsules containing the silicone components of the system. The inventors have discovered that the use of capsules having a silica shell made it possible to effectively improve the stability of the composition during storage at temperatures in the vicinity of 45°C, without harming the reactivity of its two silicone components.

More specifically, one subject of the invention is a cosmetic composition comprising, in a physiologically acceptable medium, at least one organopolysiloxane compound X having at least two alkenyl unsaturated groups, at least one organohydrogensiloxane compound Y and at least one hydrosilylation catalyst, and said compounds X and Y reacting together via a hydrosilylation reaction in the presence of the catalyst, at least one compound among the compounds X and Y being present in said composition in a form encapsulated in silica shell microcapsules, said catalyst being associated with said encapsulated compound X and/or Y or being encapsulated separately, the microcapsules being in suspension in an aqueous phase.

The composition is, in particular, intended for caring for and/or making up keratin material(s) such as the skin, lips, hair, eyelashes and nails.

The invention also relates to a cosmetic coating process for caring for and/or making up keratin material(s) comprising at least the application to said keratin material of a composition as described previously.

Within the meaning of the invention, it is understood that the composition comprises compounds X and/or Y in a form that has not yet reacted and not exclusively in the form of their hydrosilylation reaction product.

Thus, the formation of the reaction product according to the invention may either be carried out directly on the surface of the keratin material having to be treated, or initiated just before application by extemporaneous mixing of the compounds X and Y under conditions favourable to their interaction, the formation of the reaction product being, in the latter case, finalized at the surface of the keratin material. Preferably, the formation of the reaction product according to the invention is carried out directly on the surface of the keratin material having to be treated: thus, in the process described previously, preferably, the compounds X and Y react together when they are in contact with the keratin materials to be treated.

For obvious reasons, and in view of the high reactivity of the compounds X and/or Y, it is in fact necessary for their implementation to be carried out under conditions favourable to the ease of handling of the composition containing it (or them), especially in view of its spreading, for example. The process according to the invention therefore uses a composition that contains compounds X and Y, and therefore that is not set in the form of the expected final film resulting from the reaction of all of X and/or of all of Y.

As it emerges from the examples that appear below, the inventors have observed that the compositions as described previously prove to be stable over time at the temperature of around 45°C and remain effective for forming a silicone polymer film. When these compositions are spread in the form of a film over a support, for example a keratin material, the microcapsules break up under the pressure of application and also under the effect of dehydration of the film deposited and the compounds X and Y then brought into contact in the presence of the catalyst react together to form a film. Advantageously, the compositions according to the invention make it possible to defer the reaction of the compounds X and Y which takes place only when they are brought into contact after the application of the composition in the form of a film on the support in question.

According to one embodiment of the composition according to the invention, the compound X and the compound Y are each encapsulated separately in silica shell microcapsules.

According to one particularly preferred embodiment of the invention, the cosmetic composition comprises, in a physiologically acceptable medium, at least one organopolysiloxane compound X having at least two alkenyl unsaturated groups, at least one organohydrogensiloxane compound Y and at least one hydrosilylation catalyst, said compounds X and Y reacting together via a hydrosilylation reaction in the presence of the catalyst, the compounds X and Y being encapsulated separately in silica shell microcapsules, said catalyst being associated with said encapsulated compound X or said encapsulated compound Y, the microcapsules being in suspension in an aqueous phase. According to this embodiment, the compounds X and Y and also the catalyst are encapsulated in silica shell capsules.

According to the particularly preferred embodiment of the invention described previously, the cosmetic composition comprises a first portion of the microcapsules containing an organopolysiloxane compound X having at least two alkenyl unsaturated groups and a hydrosilylation catalyst (thus forming part I of a crosslinkable siloxane composition), and a second portion of the microcapsules containing an organohydrogensiloxane compound Y (forming part II of the crosslinkable siloxane composition), the compounds X and Y reacting together via a hydrosilylation reaction in the presence of the catalyst.

The silica shell microcapsules containing the siloxane compositions that can be crosslinked via a hydrosilylation reaction have good storage stability at a temperature of around 45°C, especially after storage for 2 months. These microcapsules also have a prolonged shelf life in suspension in water and good leaktightness.

According to a first embodiment of the invention, the composition according to the invention comprises a first portion of the microcapsules containing at least one of the compounds X and Y and a second portion containing the hydrosilylation catalyst.

According to a second embodiment of the invention, the aqueous suspension comprises silica shell microcapsules containing a compound chosen from the compounds X and Y, associated with the hydrosilylation catalyst.

According to a third embodiment of the invention, a particularly preferred embodiment, the composition according to the invention comprises silica shell microcapsules containing the compound X associated with the hydrosilylation catalyst and silica shell microcapsules containing the compound Y.

Thus, according to a third particularly preferred embodiment of the invention, the composition according to the invention contains a mixture of silica shell microcapsules in which a first portion of the microcapsules contains, as the core, the first part (subsequently referred to as Part I) of a crosslinkable siloxane composition containing the compound X and a second portion of the microcapsules contains the second part (subsequently referred to as Part II) of the crosslinkable siloxane composition containing the compound Y.

When the 2 parts (I and II) of the crosslinkable siloxane composition are released from the microcapsules under certain conditions (especially by breaking during application to the keratin materials and/or during the drying of the composition deposited on the keratin materials), the compounds X and Y react to form a crosslinked siloxane composition.

According to one embodiment of the invention, the crosslinkable siloxane composition encapsulated in the silica shell microcapsules may be any siloxane composition that can be crosslinked via a hydrosilylation reaction.

Such siloxane compositions that can be crosslinked via a hydrosilylation reaction involve the reaction of an organopolysiloxane compound X containing alkenyl unsaturated hydrocarbon-based groups with an organohydrogensiloxane compound Y in the presence of a hydrosilylation catalyst.

According to one embodiment of the invention, a first portion of the silica shell microcapsules contains a core comprising a first part (Part I) of the crosslinkable siloxane composition.

Part I of the crosslinkable siloxane composition contains at least two components:
a) an organopolysiloxane compound X having at least two alkenyl unsaturated groups; and
b) a hydrosilylation catalyst (component c).
   A second portion of the microcapsules comprises the second part (subsequently known as part II) of the crosslinkable siloxane composition. Part II of the crosslinkable siloxane composition contains at least one:
c) organohydrogensiloxane compound Y.

According to one embodiment, part II of the crosslinkable siloxane composition contains the compounds X and Y, which is thus a mixture of an organopolysiloxane having at least two alkenyl unsaturated groups and of an organohydrogensiloxane.

In another embodiment, the contents of compounds X and Y used in part II of the crosslinkable siloxane composition are such that the molar ratio of SiH/unsaturated group ranges from 3 to 10.

The composition according to the invention may comprise a total content of compound X ranging from 1 to 45% by weight, preferably from 1% to 30% by weight, relative to the total weight of the composition. The total content of compound Y in the composition according to the invention may range from 1 to 45% by weight, preferably from 1% to 30% by weight, relative to the total weight of the composition.

Each component is described in detail below.

### a) The organopolysiloxane having at least two alkenyl unsaturated groups (compound X)

Organopolysiloxanes are polymers containing siloxy units independently chosen from the units (R₃SiO_{0.5}), R₂SiO) (RSiO_{1.5}) or (SiO₂),
in which R may be any hydrocarbon-based group.
These units may be combined in a varied manner to form cyclic, linear or branched structures.

Advantageously, the organopolysiloxane having at least two alkenyl unsaturated groups is a non-volatile fluid.

The expression "non-volatile fluid" is understood to mean a liquid (in particular an oil) especially having a vapour pressure of less than 1.33 Pa (0.01 mmHg).

In particular, the compound X may be chosen from any organopolysiloxane comprising at least two siloxane units and having the average formula:

R²RₘSiO_{(4-m)/2}

in which:
R is a hydrocarbon-based group having from 1 to 10 carbon atoms;
R² is an alkenyl group (unsaturated aliphatic monovalent hydrocarbon-based group) containing from 2 to 12 carbon atoms (in particular from 2 to 6 carbon atoms); and
m ranges from 0 to 2.

The alkenyl group R² may be present on any monosiloxy, disiloxy or trisiloxy unit in an organopolysiloxane molecule, for example (R²R²SiO_{0.5}), (R²RSiO) or (R²SiO_{1.5}), especially in combination with other siloxy units that do not contain a substituent R², such as (R₃SiO_{0.5}), (R₂SiO), (RSiO_{1.5}) or (SiO₂) siloxy units in which R is independently a hydrocarbon-based group containing from 1 to 20 carbon atoms, alternatively an alkyl group containing from 1 to 20 carbon atoms, alternatively an alkyl group containing from 1 to 12 carbon atoms, alternatively an alkyl group containing from 1 to 6 carbon atoms, alternatively an alkyl group containing from 1 to 4 carbon atoms or alternatively a methyl group; on condition that there are at least two R² groups in the organopolysiloxane. In particular, R may be a group chosen from methyl, ethyl, propyl, butyl, hexyl, octyl, decyl, cyclohexyl, phenyl, benzyl or phenylethyl. R² may be a group chosen from vinyl, allyl, 3-butenyl, 4-pentenyl, 5-hexenyl, 7-octenyl, 9-decenyl and 10-undecenyl.

In particular, R² is a group chosen from CH₂=CH-, CH₂=CH-CH₂-, CH₂=C (CH₃) CH₂- or CH≡C-, or similar unsaturated substituted groups such as H₂C=C(CH₃)- and HC≡C(CH₃)-.

As non-limiting examples of such siloxanes which are suitable as compound X, mention may be made of:
(R₂R²SᵢO_{1/2})ᵥ(R₂SiO_{2/2})ₓ
(R₂R²SiO_{1/2})ᵥ(R₂SiO_{2/2})ₓ(R²RSiO_{2/2})_{y}
(R₂R²SiO_{1/2})ᵥ(R₂SiO_{2/2})ₓ(RSiO_{3/2})_{z}
(R₂R²SiO_{1/2})ᵥ(R₂SiO_{2/2})ₓ(RSiO_{3/2})_{z}(SiO_{4/2})_{w}
(R₂R²SiO_{1/2})ᵥ(SiO₂)_{w}(R₂SiO)ₓ
(R₃SiO_{1/2})ᵥ(R₂SiO)ₓ(R²RSiO_{2/2})_{y}
(R₃SiO_{1/2})ᵥ(R_{2S}iO)ₓ(R²RSiO)_{y}
(R₃SiO_{1/2})ᵥ(R₂SiO)ₓ(R²RSiO)_{y}(RSiO_{3/2})_{z}
(R₃SiO_{1/2})ᵥ(R₂SiO)ₓ(R²RSiO)_{y}(SiO₂)_{w}
(R₃SiO_{1/2})ᵥ(R₂SiO)ₓ(R²RSiO)_{y}(SiO₂)_{w}(RSiO_{3/2})_{z}
(R₃SiO_{1/2})ᵥ(R₂SiO)ₓ(R²SiO_{3/2})_{z}
in which v ≥ 2, w ≥ 0, x ≥ 0, y ≥ 2 and z is ≥ 0, R is a hydrocarbon-based group, and
R² is an alkenyl group as defined previously.

The component a) of part I may also be a mixture of compounds X.
The molecular weight of the compound X may vary and is not limited.
The compound X present in the microcapsule may be diluted in a solvent or a fluid silicone. Thus, the compound X alone or the mixture of compound X + solvent or fluid silicone encapsulated may have a viscosity of 25°C ranging from 1 to 10 000 mPa.s, alternatively from 50 to 1000 mPa.s, or alternatively from 100 to 1000 mPa.s.

As an example of the compound X, mention may be made of vinyldimethylsiloxy-terminated dimethylsiloxane/vinylmethylsiloxane copolymers, trimethylsiloxy-terminated polydimethylsiloxane/polymethylvinylsiloxane copolymers, trimethylsiloxy-terminated polydimethylsiloxane/polymethylhexenylsiloxane copolymers, hexenyldimethylsiloxy-terminated polydimethylsiloxane/polymethylhexenylsiloxane copolymers, trimethylsiloxy-terminated polymethylvinylsiloxane polymers, trimethylsiloxy-terminated polymethylhexenylsiloxane polymers, vinyldimethylsiloxy-terminated polydimethylsiloxanes and vinyldimethylsiloxy-terminated polydimethylsiloxane polymers,
especially having a degree of polymerization ranging from 10 to 300, or especially having a viscosity at 25°C that ranges from 10 to 1000 mPa.s.

Preferably, the compound X is chosen from vinyldimethylsiloxy-terminated polydimethylsiloxanes.

The compound X may be chosen from polydimethylsiloxanes having a vinyl functional group (vinylsiloxanes) or polydimethylsiloxanes having a hexenyl functional group (hexenylsiloxanes), such as those having the following average formulae:

CH₂=CH(Me)₂SiO[Me₂SiO]_{x'}Si(Me)₂CH=CH₂

CH₂=CH-(CH₂)₄-(Me)₂SiO[Me₂SiO]ₓSi(Me)₂-(CH₂) ₄-CH=CH₂

Me₃SiO[(Me)₂SiO]_{x'}[CH₂=CH(Me)SiO]_{x"}SiMe₃

in which Me is a methyl group;
x' ≥ 0, preferably x' ranges from 0 to 200, preferentially x' ranges from 10 to 150; and
x" ≥ 2, preferably x" ranges from 2 to 50, preferentially x" ranges from 2 to 10.

The preferred compound X is, in particular, chosen from those of the following average formula:

CH₂=CH (Me) ₂SiO [Me₂SiO] _{x'} Si (Me)₂CH=CH₂

in which Me is a methyl group;
x' ≥ 0, preferably x' ranges from 0 to 200, preferentially x' ranges from 10 to 150.

Polydimethylsiloxanes having a vinyl or hexenyl functional group are known and are available commercially, for example under the names SFD 128, DC4-2764, DC2-7891, DC2-7754, DC2-7891, and DC 2-7463, SFD-117, SFD-119, SFD 120, SFD 129, DC 5-8709, LV, 2-7038, DC 2-7892, 2-7287, 2-7463, and dihexenyl terminal DC7692, DC7697 from DOW CORNING.

### b) The hydrosilylation catalyst

The component c) is a hydrosilylation catalyst. It may be chosen from catalysts based on metals from group VIII such as catalysts based on platinum, rhodium, iridium, palladium or ruthenium.
The catalysts based on group VIII metals used for crosslinking the compositions may be any of those known for catalysing the reactions of hydrogen atoms bonded to the silicon with alkenyl groups bonded to the silicon.
Use is preferably made of a platinum-based catalyst, in particular catalysts of platinum metal or of platinum compounds, or platinum complexes.

Platinum catalysts that can be used are described in documents US 2,823,218 (that is commonly referred to as Speier's catalyst) and US 3,923,705. The platinum catalyst may be Karstedt's catalyst, which is described in patents US 3,715,334 and US 3,814,730. Karstedt's catalyst is a divinyltetramethyldisiloxane platinum complex which contains, in particular, around 1% by weight of platinum in a solvent such as toluene. According to one alternative embodiment, the platinum catalyst may be a reaction product of chloroplatinic acid and of an organosilicon compound containing terminal unsaturated aliphatic groups, as described in patent US 3,419,593. According to another embodiment, the catalyst may be a neutralized complex of platinum chloride and of divinyltetramethyldisiloxane, as described in patent US 5,175,325.

Other hydrosilylation catalysts suitable for the present invention may be, for example, rhodium catalysts such as those of formula:
[Rh(O₂CCH₃)₂]₂, Rh(O₂CCH₃)_{3,} Rh₂(C₈H₁₅O₂)₄, Rh(C₅H₇O₂)₃, Rh(C₅H₇O₂)(CO)₂, Rh(CO)[Ph₃P](C₅H₇O₂), RhX⁴₃[(R³)₂S]₃, (R²₃P)₂Rh(CO)X⁴, (R²₃P)₂Rh(CO)H, Rh₂X⁴₂Y²₄, HₐRh_{b}olefin_{c}Cl_{d}, Rh (O(CO)R³)₃₋ₙ(OH)ₙ in which X⁴ is a hydrogen, chlorine, bromine or iodine atom, Y² is an alkyl group, such as methyl or ethyl, CO, C₈H₁₄ or 0.5 C₈H₁₂, R³ is an alkyl, cycloalkyl or aryl radical, and R² is an alkyl or aryl radical or an oxygen-substituted radical, a is 0 or 1, b is 1 or 2, c is an integer ranging from 1 to 4 inclusive and d is 2, 3 or 4, and n is 0 or 1.

It is also possible to use any iridium catalyst such as Ir(OOCCH₃)₃, Ir(C₅H₇O₂)₃, [Ir(Z⁴)(En)₂]₂, or (Ir(Z⁴)(Dien)]₂, in which Z⁴ is a chlorine, bromine or iodine atom or an alkoxy group, En is an olefin and Dien is a cyclooctadiene.

Hydrosilylation catalysts are described, for example, in patents US 3,159,601; 3,220,972; 3,296,291; 3,516,946; 3,989,668; 5, 036, 117; and 5,175,325 and EP 0 347 895 B.

The hydrosilylation catalyst may be added to the composition (Part I) comprising the compound X in an amount equivalent to more than 0.001 part by weight of the platinum group metal, per one million parts (ppm) of the composition. Preferably, the concentration of the hydrosilylation catalyst in the composition is such that it is capable of providing the equivalent of at least one part per million of the platinum group metal, especially with respect to the total weight of the compound X. Typically, the catalyst concentration is such that it provides the equivalent of around 1 to 500, better still 1 to 100 parts per million of platinum group metal, especially relative to the total weight of the compound X.

### c) The organohydrogensiloxane (compound Y)

The compound Y is an organohydrogensiloxane having, on average, more than 2 hydrogen atoms bonded to the silicon per molecule.
Within the meaning of the invention, an organohydrogensiloxane denotes any organopolysiloxane containing a hydrogen atom bonded to a silicon atom (SiH).

The organohydrogensiloxanes are organopolysiloxanes having at least one siloxy unit that has at least one SiH group, so that at least one siloxy unit present in the organopolysiloxane has one of the following formulae: (R₂HSiO_{0.5}), (RHSiO) or (HSiO_{1.5}). Thus, the organohydrogensiloxanes used according to the present invention may comprise any number of siloxy units of formula: (R₃SiO_{0.5}), (R₂SiO), (RSiO_{1.5}), (R₂HSiO_{0.5}), (RHSiO), (HSiO_{1.5}) or (SiO₂), on condition that they comprise at least two siloxy units having an SiH group in the molecule.

The compound Y may be a single linear or branched organohydrogensiloxane or a mixture comprising two or more linear or branched organohydrogensiloxanes which differ over at least one of the following properties: structure, viscosity, average molecular weight, siloxane units and sequence. There is no particular restriction on the molecular weight of the organohydrogensiloxane, and typically the viscosity at 25°C may range from 3 to 10 000 mPa.s, in particular ranging from 3 to 1000 mPa.s, and better still ranging from 10 to 50 mPa.s.

The amount of SiH units present in the organohydrogensiloxane may vary, but it contains at least two SiH units per molecule of hydrogensiloxane. The content of SiH units (denoted by %SiH) is expressed as the weight percentage of hydrogen in the organohydrogensiloxane. Advantageously, the %SiH may range from 0.01 to 10% by weight, preferably from 0.1 to 5%, and better still from 0.5 to 2% of the total weight of the organohydrogensiloxane.

The organohydrogensiloxane may have the following average formula:

(R³₃SiO_{0.5})ₐ(R⁴₂SiO)_{b}(R⁴HSiO)_{c}

in which:
R³ is hydrogen or R⁴,
R⁴ is a monovalent hydrocarbon-based group having from 1 to 10 carbon atoms;
a ≥ 2;
b ≥ 0, preferably b = 1 to 500, preferentially b = 1 to 200; and
c ≥ 2, preferably c = 2 to 200, preferentially c = 2 to 100.
R⁴ may be a substituted or unsubstituted, aliphatic or aromatic, hydrocarbon-based group. As an unsubstituted aliphatic monovalent hydrocarbon-based group, mention may be made of alkyl groups such as methyl, ethyl, propyl, pentyl, octyl, undecyl and octadecyl and cycloalkyl groups such as cyclohexyl. As a substituted aliphatic hydrocarbon-based group, mention may be made of halogenated alkyl groups such as chloromethyl, 3-chloropropyl and 3,3,3-trifluoropropyl. As an aromatic hydrocarbon-based group, mention may be made of phenyl, tolyl, xylyl, benzyl, styryl and 2-phenylethyl.

According to one embodiment, the organohydrogensiloxane may contain additional siloxy units having the following average formulae:

(R³₃SiO_{0.5})ₐ(R⁴₂SiO)_{b}(R⁴HSiO)_{c}(R⁴SiO_{1.5})_{d};

(R³₃SiO_{0.5})ₐ(R⁴₂SiO)_{b}(R⁴HSiO)_{c}(SiO₂)_{d};

(R³₃SiO_{0.5})ₐ(R⁴₂SiO)_{b}(R⁴HSiO)_{c}(SiO₂)_{d}(R⁴SiO_{1.5})ₑ

or mixtures thereof,
in which:
R³ is a hydrogen atom or a group R⁴;
R⁴ is a monovalent hydrocarbon-based radical having from 1 to 10 carbon atoms; and
a ≥ 2, b ≥ 0, c ≥ 2, d ≥ 0, and e ≥ 0.

According to another embodiment, the organohydrogensiloxane may be chosen from dimethyl, methylhydrogen polysiloxanes having the average formula:

(CH₃)₃SiO[(CH₃)₂SiO]_{b}[(CH₃)HSiO]_{c}Si(CH₃)₃

in which b ≥ 0, preferably b = 1 to 200, preferentially b = 1 to 100; and
c ≥ 2, preferably c = 2 to 100, preferentially c = 2 to 50.

The processes for manufacturing organohydrogensiloxanes are well known in the art of silicones, and many organohydrogensiloxanes are commercially available.

### Preparation of the capsules:

The amounts of compound X and of compound Y used in the oily phases for preparing the separate microcapsules containing parts I and II may vary. However, the amounts used in the total siloxane composition may be adjusted in order to attain the desired molar ratio of SiH groups of the compound Y relative to the unsaturated groups present in the compound X. Typically, a sufficient amount of compound Y is used to provide a molar ratio of SiH of compound Y relative to the unsaturated alkenyl groups of compound X that is greater than 1, especially that ranges from 1 to 10, preferably that ranges from 1 to 4, and preferentially that ranges from 2 to 3.

Thus, in the composition according to the invention, the compounds X and Y may be present in contents such that the molar ratio of SiH of compound Y relative to the unsaturated alkenyl groups of compound X is greater than 1, in particular ranging from 1 to 10, preferably ranging from 1 to 4, and preferentially ranging from 2 to 3.

The aqueous suspensions of silica shell microcapsules may be prepared by any process known from the prior art.
Generally, there are two processes normally used for preparing silica shell microcapsules.

The first technique uses an in situ polymerization of a silica precursor (also known as a sol-gel process), after mixing the silica precursor with an oily phase.

Representative and non-limiting examples of the in situ polymerization process are described in documents US 6159453, US 6238650, US 6303149 and WO 2005/009604.

The second technique uses an ex·situ process in which the polymerization of the silica precursor is carried out via an emulsion polymerization process. Representative and non-limiting examples of this ex situ polymerization process are described in application WO 03/066209.

According to one embodiment, the silica shell microcapsules are prepared by:
I) mixing an oily phase containing the compound X (or part I) or the compound Y (or part II) or the hydrosilylation catalyst (if intended to be encapsulated separately) of the crosslinkable siloxane composition and of an aqueous solution of a cationic surfactant in order to form an oil-in-water emulsion;
II) addition of a silica precursor compound that reacts with water comprising a tetraalkoxysilane in the oil-in-water emulsion;
III) polymerization of the tetraalkoxysilane at the oil/water interface of the emulsion in order to form a microcapsule having a core that contains either part I or part II of the crosslinkable siloxane composition and a silica shell; and
IV) mixing the microcapsules containing the compound X or part I of the crosslinkable siloxane composition with the microcapsules containing the compound Y or part II of the crosslinkable siloxane composition and optionally with the microcapsules containing the catalyst if encapsulated separately.

According to one particularly preferred embodiment, the above process is carried out twice, a first time in order to prepare the microcapsules containing the compound X or part I of the crosslinkable siloxane composition, and a second time in order to prepare the microcapsules containing the compound Y or part II of the crosslinkable siloxane composition.
The resulting aqueous suspensions of the microcapsules are then mixed in order to form a mixture of microcapsules in aqueous suspension.

In another embodiment variant of the invention, the above process is carried out 3 times, a first time in order to prepare the microcapsules containing the compound X, a second time in order to prepare the microcapsules containing the compound Y and a third time in order to prepare the microcapsules containing the hydrosilylation catalyst.

According to the preferred embodiment of the invention, part I of the crosslinkable siloxane composition contains the compound X and the component c) described previously. Typically, part I contains the compound X and a hydrosilylation catalyst effective for carrying out the hydrosilylation reaction. For example, part I may contain 96-98% by weight of compound X and 2-4% by weight of a solution of platinum catalyst (containing 18 ppm of platinum) as component c).

According to the preferred embodiment of the invention, part II of the crosslinkable siloxane composition contains at least the compound Y. Part II may contain additional components, as described below. According to one embodiment, part II of the crosslinkable siloxane composition advantageously contains the compounds X and Y, and therefore contains a mixture of the organopolysiloxane having at least two unsaturated groups and of the organohydrogensiloxane.

When the compounds X and Y are mixed to form part II, the amounts may vary, depending on the objectives desired for the crosslinking of the siloxane composition and on the properties of the film obtained (mechanical properties). Typically, the amounts may range from 50-94% by weight of compound X and from 6 to 50% by weight of compound Y.

In other embodiment, the amounts of compounds X and Y used in part II of the crosslinkable siloxane composition are such that the molar ratio of SiH/unsaturated group may range from 3 to 10, and preferably from 4 to 9, and preferentially may range from 5 to 7. Such ratios make it possible to provide crosslinkable siloxane compositions that crosslink by forming thin films, while having good storage stability in aqueous suspension.

The optimized ratio in this embodiment makes it possible to obtain an excellent degree of crosslinking of the siloxane compositions, while maintaining storage stabilities of such compositions in an aqueous medium.

In the present invention, the expression "oily phase" includes the compound X or part I or the compound Y or part II of the crosslinkable siloxane composition or else the hydrosilylation catalyst and an additional oil. Typically, the oily phase is a liquid during the preparation of the oil-in-water emulsion. The oily phase may contain additional hydrocarbon or silicone or fluorinated oils, and preferably additional silicone oils, as a mixture with part I or part II.

The oily phase containing part I or II of the crosslinkable siloxane composition may contain other ingredients, in particular hydrocarbon-based or silicone-based ingredients, which are substantially soluble at ambient temperature (25°C) with the components of the other oily phase, and conversely are substantially insoluble in water. Such additional ingredients may be chosen from volatile silicones, fluid polydimethylsiloxanes, siloxanes having a weight-average molecular weight greater than 1000 (including silicone elastomers and resins), hydrocarbon oils, waxes and emollients.

The oily phase containing either the compound X or Part I or the compound Y or Part II of the crosslinkable siloxane composition or else the hydrosilylation catalyst may contain other known ingredients such as agents that promote the bursting of the capsules. The expression "agents that promote the bursting of the capsules" encompasses any ingredient, or mixtures of ingredients, added to the oily phase for the purpose of initiating the controlled release of the encapsulated core material, especially during the dehydration of the film of the composition deposited on the keratin materials.
The agents that promote the bursting of the capsules may be chosen from volatile hydrophobic hydrocarbon or silicone compounds. The agents that promote the bursting of the capsules may especially be chosen from volatile branched hydrocarbon compounds such as isohexane, isoheptane, isooctane, isodecane, and isododecane; volatile linear siloxanes such as, for example, hexamethyldisiloxane and decamethyltetrasiloxane; volatile cyclic siloxanes such as octamethylcyclotetrasiloxane, decamethylcyclopentasiloxane and dodecylmethylcyclohexasiloxane.

The oily phase containing either the compound X or Part I or the compound Y or Part II of the crosslinkable siloxane composition or else the hydrosilylation catalyst is advantageously mixed with an aqueous solution of cationic surfactants in order to form an oil-in-water emulsion.

Cationic surfactants that can be used according to the invention may be quaternary ammonium hydroxides such as octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, didodecyldimethylammonium hydroxide, dioctadecyldimethylammonium hydroxide, tallow trimethylammonium hydroxide and cocotrimethylammonium hydroxide, and also salts thereof, fatty amines and salts thereof, amides of fatty acids and salts thereof, pyridinium compounds, quaternary ammoniums of benzimidazolines and polypropanol polyethanol amines, without being limited to this list of cationic surfactants. As the preferred cationic surfactant, use is made of cetyltrimethylammonium chloride or bromide.

The cationic surfactant may also be chosen from amphoteric surfactants such as cocamidopropyl betaine, cocamidopropyl hydroxysulphate, cocobetaine, sodium cocamidoacetate, cocodimethyl betaine, N-coco-3-aminobutyric acid, but are not limited to this list of amphoteric surfactants.

The surfactants described previously may be used alone or as mixtures. The cationic or amphoteric surfactant is dissolved in water and the resulting aqueous solution is used as a component of the oil-in-water emulsion of step I).

Without being bound to any theory, the use of the cationic or amphoteric surfactant favours the condensation and the polymerization of the tetraalkoxysilane, as described previously, at the interface of the droplets of the emulsified oily phase, resulting in porous microcapsules. The tetraalkoxysilane hydrolyses and condenses by reaction in the emulsion. The anionic charge of the hydrolysis product is attracted by the cationic or amphoteric surfactant to the interface where the silica-based polymer shell forms.

The concentration of the cationic surfactant during the formation of the oil-in-water emulsion may range from 0.1% to 0.3% by weight relative to the weight of the oily phase used. Typically, the use of a small amount of cationic or amphoteric surfactant during the emulsification of the oily phase and the reaction of the alkoxysilane results in microcapsules which are more resistant to the diffusion or to the release of the oily phase from the microcapsules.

Additional surfactants, and in particular anionic surfactants, may be added during the formation of the oil-in-water emulsion. Suitable anionic surfactants are, for example, polyoxyalkylenated alkyl ethers such as polyethylene glycol and alkyl (C12-C14) ethers, polyoxyalkylenated sorbitan ethers, polyoxyalkylenated alkoxylate esters, polyoxyalkylenated alkylphenol esters, ethylene glycol/propylene glycol copolymers, polyvinyl alcohol and alkylpolysaccharides, for example such as those described in patent US 5,035,832, but without being limited to this list of nonionic surfactants.

The aqueous solution of cationic or amphoteric surfactant may contain additional water-soluble ingredients, such as water-miscible organic solvents, for instance ethanol. Other water-soluble ingredients may be added to the aqueous phase that are normally used in care formulations. Such ingredients are, in particular, additional surfactants, thickeners, preservatives, antimicrobial agents, water-soluble active agents and fragrances.

The oily phase and the aqueous solution of cationic or amphoteric surfactant are mixed to form an oil-in-water emulsion. The mixing and the formation of the emulsion may be carried out using any known emulsion technique. Typically, the oily phase and the aqueous solution of cationic or amphoteric surfactant are mixed using simple mixing techniques in order to form an emulsion. The size of the oil droplets of the emulsion may be reduced before the addition of tetraalkoxysilane by any equipment of the art of emulsions. The equipment used for the emulsification may be a high-pressure homogenizer, an ultrasound device, rotor-stator agitators, a colloidal mill, a microfluidizer, blades, propellers and the combination thereof, without being limited to this list of equipment. This supplementary process step reduces the size of the initial oil-in-water cationic emulsion particles to values ranging from 0.2 to 500 micrometres, and preferably ranging from 0.5 to 100 micrometres.

The weight ratio between the oily phase containing either part I or part II of the crosslinkable siloxane composition and the aqueous phase in the emulsion may range from 40:1 to 1:50, in particular during the formulation of the suspension of microcapsules. Usually, the weight ratio between the oily phase and the aqueous phase ranges from 2:1 to 1:3. If the oily phase is very viscous, a phase inversion process may be used in which the oily phase is mixed with the surfactant and a small amount of water, for example from 2.5 to 10% by weight of water relative to the weight of the oily phase, in order to form a water-in-oil emulsion which inverts to an oil-in-water emulsion when it is agitated. Supplementary water may be added in order to dilute the emulsion to the desired concentration.

According to one embodiment, the density of the oily phase compared to that of the aqueous phase in the emulsion is approximately identical, so that the densities may be equal or, alternatively, may differ by 2%, or else by 1%, or even by 0.5%.

The second and third steps of the process involve the addition of a water-reactive silicone compound comprising a tetraalkoxysilane, having alkoxy groups comprising from 1 to 4 (preferably from 1 to 2) carbon atoms, to the oil-in-water emulsion, and the polymerization of the tetraalkoxysilane at the oil/water interface of the emulsion. Without being bound by any theory, the third step of the process performs an ex situ emulsion polymerization in which the tetraalkoxysilane precursor hydrolyses and condenses at the oil/water interface resulting in the formation of core/shell microcapsules via the phase transfer of said precursor.

The tetraalkoxysilane, such as tetraethoxysilane (TEOS), may be used in the form of a liquid partial condensate (also known as an oligomer) or monomer. The tetraalkoxysilane may be used in combination with one or more silicon compound(s) that react(s) with water having at least two, preferably at least 3, Si-OH groups or hydrolysable groups bonded to the silicon, for example an alkyltrialkoxysilane such as methyltrimethoxysilane or a liquid condensate (or oligomer) of an alkyltrialkoxysilane or of a (substituted alkyl)trialkoxysilane. Hydrolysable groups may be, for example, alkoxy or acyloxy groups bonded to the silicon. The water-reactive silicon compound may, for example, comprise from 50 to 100% by weight of tetraalkoxysilane and from 0 to 50% by weight of trialkoxysilane. The alkyl and alkoxy groups of the tetraalkoxysilanes or of other silanes preferably comprise from 1 to 4 carbon atoms, and preferentially from 1 to 2 carbon atoms. The tetraalkoxysilane, and other water-reactive silicon compound if used, hydrolyses and condenses to form a polymer as a three-dimensional network of a silica-based material, around emulsified droplets of part I or II of the crosslinkable siloxane composition. The water-reactive silicon compound typically comprises at least 75%, especially from 90-100% by weight of tetraalkoxysilane. The tetraalkoxysilane provides the shell of the impermeable microcapsules, forming a three-dimensional network that is substantially made up of SiO_{4/2} units. The shell thus formed is constituted of silica.

The water-reactive silicon compound may also comprise an alkoxysilane having other organofunctional groups such as a quaternized substituted alkyl group. A preferred type of quaternized alkoxysilane has the general formula:

(CH₃O)₃SiCH₂CH₂CH₂N⁺(CH₃)₂(CH₂)₁₇CH₃Cl⁻.

The water-reactive silicon compound may, for example, comprise from 10-100% by weight of tetraalkoxysilane and from 0-90% by weight of trialkoxysilane. Mixtures of quaternized aminoalkyltrialkoxysilanes with the tetraalkoxysilane are particularly effective for encapsulating the silicone composition described previously.

The tetraalkoxysilane, and other silicon compounds if used, may be added to the emulsion of composition of water-reactive material as an undiluted liquid or as a solution in an organic solvent or in an emulsion.

The tetraalkoxysilane and the oil-in-water emulsion are mixed during their addition. The tetraalkoxysilane in water reacts and polymerizes to form the silicon-based polymer shell at the surface of the emulsified droplets. The mixing is typically carried out with agitation techniques. Standard agitation techniques are sufficient to maintain the size of the initial oil-in-water emulsion particles while enabling the tetraalkoxysilane to polymerize and to condense at the oil/water interface.

The amount of tetraalkoxysilane added in step II may range from 6/1 to 1/13, preferably from 1.2/1 to 1/7.3, preferentially from 1.3 to 1/6.1, relative to the total weight of the oily phase present in the emulsion.

The polymerization of the water-reactive silicon compound at the oil/water interface is a condensation reaction which may be carried out at acid, neutral or basic pH.
The condensation reaction is generally conducted at ambient temperature and ambient pressure, but may be conducted at a higher temperature, for example up to 95°C, and at a lower or higher pressure, for example under vacuum in order to eliminate the volatile alcohol produced during the condensation reaction.

The presence of colloidal silica particles in the aqueous suspension of silica shell microcapsules may limit the storage stability of the microcapsules. These colloidal silica particles may be considered to be a reaction by-product formed during the polymerization of the tetraalkoxysilane during the manufacture of the silica microcapsules. The stability of the present aqueous suspension of silica shell microcapsules may be improved by reducing the amount of colloidal silica particles in the aqueous suspension or, alternatively, by inhibiting the colloidal silica particles by addition of a sequestrant of these colloidal particles. The expression "colloidal silica sequestrant" is understood to mean any compound or material which, when added to the suspension of silica shell microcapsules that also contains colloidal silica particles, interacts with the colloidal particles in such a way that it prevents the reaction thereof or the coagulation thereof. The techniques for removing the colloidal silica particles and various colloidal silica sequestrants are described in application US 61/096397.

The composition according to the invention may comprise a colloidal silica sequestrant which may be any organofunctional silane. According to one embodiment, the organofunctional silane may be a quaternized trialkoxysilane, such as for example the cetrimoniumpropyltrimethoxysilane chloride sold under the name Dow Corning® Q9-6346 by Dow Corning.

The colloidal silica sequestrant may be a silicone polyether, such as for example those sold under the names Dow Corning® 190, 193 and 2-5657 by Dow Corning.

The microcapsules of the composition according to the invention may have a number-average size ranging from 0.5 to 100 µm, preferably ranging from 1 to 50 µm.

As stated previously, a composition in accordance with the invention comprises a physiologically acceptable medium.

The expression "physiologically acceptable medium" is intended to denote a medium that is particularly suitable for applying a composition according to the invention to a keratin material such as the skin or the lips. The physiologically acceptable medium is generally suited to the nature of the support onto which the composition composition must be applied, and also to the form in which the composition is intended to be packaged.

The physiologically acceptable medium may comprise an aqueous phase, which may essentially comprise water.

It may also comprise a mixture of water and of water-miscible solvent (miscibility with water of greater than 50% by weight at 25°C), for instance one, or a mixture of, lower monoalcohol(s) containing from 1 to 5 carbon atoms, such as ethanol or isopropanol, glycols containing from 2 to 8 carbon atoms, such as propylene glycol, ethylene glycol, 1,3-butylene glycol or dipropylene glycol, and mixtures thereof.

The aqueous phase (water and optionally the water-miscible solvent) may be present in a content ranging from 5% to 95% by weight, preferably from 10% to 85% by weight and better still from 20% to 80% by weight relative to the total weight of the composition.

The physiologically acceptable medium may also comprise a liquid fatty phase, comprising one or more volatile or non-volatile oils, and/or a solid fatty phase comprising one or more waxes and/or pasty compounds, and a mixture thereof.

For the purposes of the patent application, the expression "liquid fatty phase" means a fatty phase that is liquid at room temperature (25°C) and atmospheric pressure (760 mmHg), composed of one or more non-aqueous fatty substances that are liquid at room temperature, also known as oils or organic solvents.

The oil may be chosen from volatile oils and/or non-volatile oils, and mixtures thereof.

The oil(s) may be present in a content ranging from 1% to 90% by weight and preferably from 5% to 50% by weight relative to the total weight of the composition.

These oils may be hydrocarbon-based oils, silicone oils or fluorinated oils, or mixtures thereof.

The expression "hydrocarbon-based oil" is understood to mean an oil that mainly contains hydrogen and carbon atoms and optionally oxygen, nitrogen, sulphur and phosphorus atoms. The hydrocarbon-based oils may be chosen from hydrocarbon-based oils containing from 8 to 16 carbon atoms, and especially branched C₈-C₁₆ alkanes, for instance C₈-C₁₆ isoalkanes of petroleum origin (also known as isoparaffins), for instance isododecane (also known as 2,2,4,4,6-pentamethylheptane), isodecane, isohexadecane and, for example, the oils sold under the trade names Isopar^{®} and Permethyl^{®}, C₈-C₁₆ branched esters and isohexyl neopentanoate, and mixtures thereof.

Hydrocarbon-based oils that may also be mentioned include:
hydrocarbon-based oils of plant origin, such as triesters of fatty acids and of glycerol, the fatty acids of which may have varied chain lengths from C₄ to C₂₄, these chains possibly being linear or branched, and saturated or unsaturated; these oils are especially wheatgerm oil, sunflower oil, grapeseed oil, sesame seed oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppyseed oil, pumpkin oil, marrow oil, blackcurrant oil, evening primrose oil, millet oil, barley oil, quinoa oil, rye oil, safflower oil, candlenut oil, passionflower oil or musk rose oil; or caprylic/capric acid triglycerides,
for instance those sold by the company Stéarineries Dubois or those sold under the names Miglyol 810, 812 and 818 by the company Dynamit Nobel;
   - synthetic ethers containing from 10 to 40 carbon atoms;
   - apolar hydrocarbon-based oils, for instance squalene, linear or branched hydrocarbons such as liquid paraffin, liquid petroleum jelly and naphthalene oil,
hydrogenated or partially hydrogenated polyisobutene, isoeicosane, squalane, decene/butene copolymers and polybutene/polyisobutene copolymers, especially Indopol L-14, and polydecenes such as Puresyn 10, and mixtures thereof;
   - synthetic esters, for instance oils of formula R₁COOR₂ in which R₁ represents a linear or branched fatty acid residue containing from 1 to 40 carbon atoms and R₂ represents a hydrocarbon-based chain, which is especially branched, containing from 1 to 40 carbon atoms, on condition that R₁ + R₂ ≥ 10, for instance Purcellin oil (cetostearyl octanoate), isopropyl myristate, isopropyl palmitate, C₁₂ to C₁₅ alcohol benzoate, hexyl laurate, diisopropyl adipate, isononyl isononanoate, 2-ethylhexyl palmitate, isostearyl isostearate, alcohol or polyalcohol octanoates, decanoates or ricinoleates, for instance propylene glycol dioctanoate; hydroxylated esters, for instance isostearyl lactate or diisostearyl malate; and pentaerythritol esters;
   - fatty alcohols that are liquid at room temperature with a branched and/or unsaturated carbon-based chain containing from 12 to 26 carbon atoms, for instance octyldodecanol, isostearyl alcohol, oleyl alcohol, 2-hexyldecanol, 2-butyloctanol or 2-undecylpentadecanol;
   - higher fatty acids such as oleic acid, linoleic acid or linolenic acid;
   - and mixtures thereof.

As silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

The silicone oils may also be:
- polydimethylsiloxanes (PDMS),
- polydimethylsiloxanes comprising alkyl or alkoxy groups, which are pendent and/or at the end of a silicone chain, these groups each containing from 3 to 40 carbon atoms,
- phenyl silicones, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates;
- and mixtures thereof.

The compositions according to the invention may also comprise at least one fatty substance that is solid at room temperature, especially chosen from waxes and pasty fatty substances, and mixtures thereof. These fatty substances may be of animal, plant, mineral or synthetic origin.

The compositions according to the invention may also contain ingredients commonly used in cosmetics, such as oils, waxes, pasty fatty substances, vitamins, thickeners, gelling agents, trace elements, softeners, sequestrants, fragrances, basifying or acidifying agents, preservatives, surfactants, antioxidants, fibres, fillers, dyestuffs, film-forming polymers, cosmetic active agents such as bactericidal active agents or antiperspirants, neutralizers, emollients, moisturizers, and mixtures thereof.

Needless to say, a person skilled in the art will take care to select this or these optional additional compound(s), and/or the amount thereof, such that the advantageous properties of the corresponding composition according to the invention are not, or are not substantially, adversely affected by the envisaged addition.

The compositions according to the invention may be, independently, in the form of a suspension, a dispersion, a solution, a gel, an emulsion, especially an oil-in-water (O/W), wax-in-water or water-in-oil (W/O) emulsion, or a multiple emulsion (W/O/W or polyol/O/W or O/W/O), or in the form of a cream, a paste, a mousse, a dispersion of vesicles, especially of ionic or nonionic lipids, a two-phase or multiphase lotion, or a paste, especially a soft paste.

The compositions according to the invention or used for the process according to the invention may be in the form of a composition for protecting, treating or caring for the face, the hands, the feet, the major anatomical folds or the body (for example a day cream, a night cream, a makeup-removing cream, an antisun composition, a protective or caring body milk, an after-sun milk, a skincare lotion, gel or mousse, or an artificial tanning composition); an aftershave composition.

They may also be used for making up the skin, the lips, the eyelashes and/or the nails depending on the nature of the ingredients used.

In particular, the compositions according to the invention may be, independently, in the form of a foundation, a lip product, especially a lipstick, a concealer product, an eye contour product, an eyeliner, a mascara, an eyeshadow, a body makeup product or a skin colouring product.

According to one embodiment, the compositions are compositions for coating bodily or facial skin, more particularly bodily or facial skin makeup or care compositions, for instance foundations, or body makeup compositions.

A person skilled in the art may select the appropriate galenic form, and also the method for preparing it, on the basis of his general knowledge, taking into account, on the one hand, the nature of the constituents used, especially their solubility in the support, and, on the other hand, the intended use of each composition.

The composition according to the invention may be used as a finishing composition (also known as a topcoat) to be applied on top of a first deposition formed by prior application of a first cosmetic composition on keratin materials.

Thus, according to one embodiment of the cosmetic process for caring for or making up keratin materials according to the invention, the composition according to the invention is applied after the prior formation of a first deposition on the keratin materials obtained by applying a first cosmetic composition to the keratin materials. The first cosmetic composition comprises, for example and as is usual, a cosmetic ingredient as described previously.

The invention is illustrated in greater detail by the examples described below. Unless otherwise stated, the amounts indicated are expressed as weight percentages.

### Example 1:

The systems of reactive silicone components below were used:
Ingredients of the composition Part I (catalyst mixture)

| **Component** | **Chemical name** | **Abbreviated name** |
|---|---|---|
| a) | Dimethylvinylsiloxy-terminated polydimethylsiloxane | Vinyl siloxane |
| | Viscosity = 300-600 mPa.s (cP) at 25°C | |
| | Vinyl functionalization: 0.45% (weight/weight) | |
| b) | Karstedt's catalyst (CAS Registry No. 684789-22) | Catalyst |
| | 1,3-Diethenyl-1,1,3,3-tetramethyldisiloxane complex of platinum dispersed in *VINYL SILOXANE,* containing 0.52% by weight of elementary Pt | |

Ingredients of composition Part II - (base mixture)

| **Component** | **Chemical name** | **Abbreviated name** |
|---|---|---|
| a) | Dimethylvinylsiloxy-terminated polydimethylsiloxane | Vinyl siloxane |
| | Viscosity = 300-600 mPa.s (cP) | |
| | Vinyl functionalization: 0.45% | |

| | | |
|---|---|---|
| | (weight/weight) | |
| c) | Trimethylsiloxy-terminated dimethylmethylhydrogensiloxane | SiH siloxane |
| | Viscosity = 5 mPa.s (cP) at 25°C | |
| | SiH functionalized: 0.795% | |

The viscosity of the vinyl siloxane and SiH siloxane was measured at 23°C according to the Dow Corning CTM 0050 method using a Brookfield rotating viscometer with an RVF #2 spindle at 20 rpm.
a) The following 2 aqueous suspensions of microcapsules were prepared:
   Suspension A:
   A suspension of microcapsules containing part I of the crosslinkable siloxane composition was prepared by dissolving 3.35 g of cetyltrimethylammonium chloride (CCTA) in 791.9 g of water. Then a mixture of 675.6 g of vinyl siloxane (component a) and of 21 g of catalyst was added to the CCTA/water mixture in order to form an oil-in-water emulsion, using an Ultra-Turrax T25 Basic machine for 180 seconds at 9500 rpm. The emulsion was then agitated using an APV 1000 homogenizer at a pressure of 700 bar in order to produce a fine emulsion having a volume-average particle size (Dv 0.9) of less than 15 µm. The pH of the emulsion was adjusted to 3.7 by addition of 2.5M of HCl.
      Then 12.86% by weight (relative to the total weight of the emulsion) of tetraethylorthosilicate (TEOS) was added while mixing at 400 rpm for 4 hours. After complete hydrolysis and condensation of the TEOS, an aqueous suspension of core/shell microcapsules was obtained having a volume-average size (Dv 0.5) of 4.7 µm. The aqueous suspension was diluted with water in order to obtain a solids content of 30%. Then, at the end, 0.3% of 3-(trimethoxysilyl)propyldimethylhexadecylammonium chloride was added to the suspension in order to avoid thickening at 45°C.
   Suspension B:
   A suspension of microcapsules containing part II of the crosslinkable siloxane composition was prepared by dissolving 3.35 g of cetyltrimethylammonium chloride (CCTA) in 813.5 g of water. Then 600 g of vinyl siloxane (component a) and 75 g of SiH siloxane (component c) were added to the CCTA/water mixture with agitation at 400 rpm in order to form an oil-in-water emulsion. Next, the emulsion was agitated using an Ultra-Turrax T25 Basic machine for 90 seconds at 9500 rpm. The emulsion was then subjected to additional shearing using an APV 1000 homogenizer at a pressure of 700 bar in order to produce a fine oil-in-water emulsion having a volume-average particle size (Dv 0.9) of less than 15 µm. The pH of the emulsion was adjusted to 4.7 by addition of 2.5 M HCl. Then, 12.86% by weight (relative to the total weight of the emulsion) of tetraethylorthosilicate (TEOS) were added with agitation at 400 rpm for 4 hours. After complete hydrolysis and condensation of the TEOS, an aqueous suspension of core/shell capsules was obtained, having a volume-average particle size (Dv 0.5) of 3.6 µm. The suspension was diluted with water in order to obtain a solids content of 30%. Then, in the end, 0.3% of 3-(trimethoxysilyl)propyldimethylhexadecylammonium chloride were added to the suspension in order to prevent thickening at 45°C.
b) The two aqueous suspensions A and B containing the microcapsules of the catalyst and base mixtures were then mixed in a 1/1 weight ratio in order to thus obtain an aqueous suspension C. The mixture thus obtained was kept for 2 months at 45°C: after storage it was observed that the aqueous suspension is still liquid, with no setting: the microcapsules remain leaktight and therefore have good properties of encapsulation of the reactive silicone components.

By spreading the aqueous suspension C over a glass plate, it was observed that after evaporation of the water a film was obtained resulting from the reaction of the reactive silicone components released from the microcapsules that were broken during the spreading of the suspension C on the glass plate.

### Example 2:

A facial care serum was prepared having the following composition:

| | | |
|---|---|---|
| Aqueous suspension C of microcapsules | | |
| according to Example 1 | | 15 g |
| Glycerol | | 3 g |
| Butylene glycol | | 2 g |
| Sucrose stearate (Crodesta F110 from Croda) | | 1 g |
| Cyclohexasiloxane | | 5 g |
| Oxyethylenated (20 EO) methylglucose | | |
| (Glucam E20 from Lubrizol) | | 0.1 g |
| Hydroxyethyl cellulose | | 0.1 g |
| Water | q.s. for | 100 g |

The serum applied to the skin spreads easily and results, after reaction of the silicone components released from the microcapsules, in a film that perfectly matches the skin relief.

### Example 3:

A foundation was prepared having the following composition:
- Dimethicone (Dow Corning 200 Fluid 350 cst from Dow Corning) 5 g
- Glycerol 5 g
- Xanthan gum 0.2 g
- Glycerol stearate (Witconol RHT from Witco) 1.1 g
- PDMS-coated pigments 12 g
- Aqueous suspension C of microcapsules according to Example 1 30 g
- Water q.s. for 100 g

The foundation applied to the skin spreads easily and results, after reaction of the silicone components released from the microcapsules, in a film that perfectly matches the skin relief and that has a good transfer-resistance property.

### Example 4:

A shampoo was prepared having the following composition:

| | | |
|---|---|---|
| Sodium lauryl ether sulphate containing 2.2 mol of ethylene oxide; 70% AM | | 15 g |
| Coco betaine; 30% AM | | 3 g |
| Isopropyl myristate | | 2 g |
| Carboxyvinyl polymer (Carbopol 980) | | 0.8 g |
| Aqueous suspension C of microcapsules | | |
| according to Example 1 | | 10 g |
| Fragrance, preservative | q.s. | |
| Hydrochloric acid | q.s. pH 5-5.6 | |
| Water | q.s. for | 100 g |

Hair washed with this shampoo has a coating of the hair fibre that gives the hair a pleasant feel.

### Example 5: Two-layer foundation

A first foundation composition A was prepared comprising the following ingredients:
- Dimethicone (Dow Corning 200 Fluid 350 cst from Dow Corning) 5 g
- Glycerol 5 g
- Xanthan gum 0.2 g
- Glycerol stearate (Witconol RHT from Witco) 1.1 g
- PDMS-coated pigments 12 g
- Aqueous suspension C of microcapsules 30 g
- Water q.s. for 100 g

A second foundation composition B was prepared comprising the following ingredients:
- Dimethicone (Dow Corning 200 Fluid 350 cst from Dow Corning) 5 g
- Glycerol 5 g
- Xanthan gum 0.2 g
- Glycerol stearate (Witconol RHT from Witco) 1.1 g
- PDMS-coated pigments 12 g
- Aqueous suspension C of microcapsules according to Example 1 30 g
- Water q.s. for 100 g

The first foundation composition A is applied to the face. After drying, the second composition B is applied to the make-up.
After drying, a foundation is obtained that has good transfer-resistance properties by virtue of the film obtained by the crosslinking on the skin of the encapsulated silicone components of composition B.

## Claims

1. Cosmetic composition comprising, in a physiologically acceptable medium, at least one organopolysiloxane compound X having at least two alkenyl unsaturated groups, at least one organohydrogensiloxane compound Y and at least one hydrosilylation catalyst, and said compounds X and Y reacting together via a hydrosilylation reaction in the presence of the catalyst, at least one compound among the compounds X and Y being present in said composition in a form encapsulated in silica shell microcapsules, said catalyst being associated with said encapsulated compound X and/or Y or being encapsulated separately, the microcapsules being in suspension in an aqueous phase.

2. Composition according to Claim 1, containing at least one compound X or one compound Y in an encapsulated form.

3. Composition according to Claim 1 or 2, in which the compounds X and Y are both present in separate encapsulated forms.

4. Composition according to any one of the preceding claims, in which a first portion of the microcapsules comprises the compound X and the catalyst and a second portion comprises the compound Y, optionally associated with the compound X.

5. Composition according to any one of the preceding claims, in which the compound X is chosen from the organopolysiloxanes comprising at least two siloxane units and having the average formula:
R²RₘSiO_{(4-m)/2}
in which:
R is a hydrocarbon-based group having from 1 to 10 carbon atoms;
R² is an alkenyl group containing from 2 to 12 carbon atoms; and
m ranges from 0 to 2.

6. Composition according to any one of the preceding claims, in which the compound X is chosen from the following compounds:
CH₂=CH(Me)₂SiO[Me₂SiO]_{x'}Si(Me) ₂CH=CH₂
CH₂=CH-(CH₂)₄-(Me)₂SiO[Me₂SiO]_{x'}Si(Me)₂-(CH₂)₄-CH=CH₂
Me₃SiO[(Me)₂SiO]_{x'}[CH₂=CH(Me)SiO]ₓ"SiMe₃
in which Me is a methyl group;
x' ≥ 0, preferably x' ranges from 0 to 200, preferentially x' ranges from 10 to 150; and
x " ≥ 2, preferably x " ranges from 2 to 50, preferentially x " ranges from 2 to 10.

7. Composition according to any one of the preceding claims, in which the preferred compound X is, in particular, chosen from those of the following average formula:
CH₂=CH (Me) ₂SiO [Me₂SiO]_{x'} Si (Me) ₂CH=CH₂
in which Me is a methyl group;
x' ≥ 0, preferably x' ranges from 0 to 200, preferentially x' ranges from 10 to 150.

8. Composition according to any one of the preceding claims, in which the organohydrogensiloxane may have the following average formula:
(R³₃SiO_{0.5})ₐ(R⁴₂SiO)_{b}(R⁴HSiO)_{c}
in which:
R³ is hydrogen or R⁴,
R⁴ is a monovalent hydrocarbon-based group having from 1 to 10 carbon atoms;
a ≥ 2;
b ≥ 0, preferably b = 1 to 500, preferentially b = 1 to 200; and
c ≥ 2, preferably c = 2 to 200, preferentially c = 2 to 100.

9. Composition according to any one of the preceding claims, in which the organohydrogensiloxane may be chosen from dimethyl methylhydrogen polysiloxanes having the average formula:
(CH₃)₃SiO[(CH₃)₂SiO]_{b}[(CH₃)HSiO]_{c}Si(CH₃)₃
in which b ≥ 0, preferably b = 1 to 200, preferentially b = 1 to 100; and
c ≥ 2, preferably c = 2 to 100, preferentially c = 2 to 50.

10. Composition according to any one of the preceding claims, in which the compounds X and Y are present in contents such that the molar ratio of SiH of compound Y relative to the unsaturated alkenyl groups of compound X is greater than 1, in particular ranging from 1 to 10, preferably ranging from 1 to 4, and preferentially ranging from 2 to 3.

11. Composition according to any one of the preceding claims, in which the hydrosilylation catalyst is a platinum group metal present at a concentration of 1 to 500 parts per million, relative to the total weight of compound X.

12. Composition according to any one of the preceding claims, **characterized by** the fact that it comprises cetrimoniumpropyltrimethoxysilane chloride.

13. Composition according to any one of the preceding claims, **characterized by** the fact that it comprises a cosmetic ingredient chosen from oils, waxes, pasty fatty substances, vitamins, thickeners, gelling agents, trace elements, softeners, sequestrants, fragrances, basifying or acidifying agents, preservatives, fragrances, surfactants, antioxidants, fibres, fillers, dyestuffs, film-forming polymers, cosmetic active agents such as bactericidal active agents or antiperspirants, neutralizers, emollients, moisturizers, and mixtures thereof.

14. Cosmetic coating process for caring for and/or making up keratin material(s) comprising at least the application to said keratin material of a composition according to any one of the preceding claims.

15. Process according to the preceding claim, in which the compounds X and Y react together when they are in contact with the keratin material to be treated.

16. Process according to either of Claims 14 and 15, in which the composition comprising the microcapsules is applied on top of a first deposition formed by prior application on to the keratin materials of a first cosmetic composition.

## Patentansprüche

1. Kosmetische Zusammensetzung, die in einem physiologisch unbedenklichen Medium mindestens eine Organopolysiloxanverbindung X mit mindestens zwei ungesättigten Alkenylgruppen, mindestens eine Organohydrogensiloxanverbindung Y und mindestens einen Hydrosilylierungskatalysator umfasst, wobei die Verbindungen X und Y durch eine Hydrosilylierungsreaktion in Anwesenheit des Katalysators miteinander reagieren und wobei mindestens eine Verbindung unter den in der Zusammensetzung vorliegenden Verbindungen X und Y in einer in Mikrokapseln mit Siliciumdioxidschale verkapselten Form vorliegt, wobei der Katalysator mit der verkapselten Verbindung X und/oder Y assoziiert oder separat verkapselt ist, wobei die Mikrokapseln in einer wässrigen Phase suspendiert sind.

2. Zuaammensetzung nach Anspruch 1, die mindestens eine Verbindung X oder eine Verbindung Y in verkapselter Form enthält.

3. Zusammensetzung nach Anspruch 1 oder 2, wobei die Verbindungen X und Y beide in separaten verkapselten Formen vorliegen.

4. Zuaammensetzung nach einem der vorhergehenden Ansprüche, wobei ein erster Teil der Mikrokapseln die Verbindung X und den Katalysator umfasst und ein zweiter Teil die Verbindung Y, gegebenenfalls in Assoziation mit der Verbindung X, umfasst.

5. Zusammensetzung nach einem der vorhergehenden Ansprüche, wobei die Verbindung X aus den Organopolysiloxanen mit mindestens zwei Siloxaneinheiten und der durchschnittlichen Formel :
R²RₘSiO₍₄₋ₘ)/₂
worin :
R für eine auf Kohlenwasserstoff basierende Gruppe mit 1 bis 10 Kohlenstoffatomen steht;
R² für eine Alkenylgruppe mit 2 bis 12 Kohlenstoffatomen steht und
m im Bereich von 0 bis 2 liegt;
ausgewählt ist.

6. Zusammensetzung nach einem der vorhergehenden Ansprüche, wobei die Verbindung X aus den folgenden Verbindungen ausgewählt ist :
CH₂=CH(Me)₂SiO[Me₂SiO]_{x'}Si(Me)₂CH=CH₂
CH₂=CH-(CH₂)₄-(Me)₂SiO[Me₂SiO]_{x'}Si(Me)₂-(CH₂)₄-CH=CH₂
Me₃SiO[(Me)₂SiO]_{x'}[CH₂=CH(Me)SiO]_{x"}SiMe₃
worin Me für eine Methylgruppe steht;
x' ≥ 0, x' vorzugsweise im Bereich von 0 bis 200 liegt, x' bevorzugt im Bereich von 10 bis 150 liegt; und
x" ≥ 2, x" vorzugsweise im Bereich von 2 bis 50 liegt, x" bevorzugt im Bereich von 2 bis 10 liegt.

7. Zusammensetzung nach einem der vorhergehenden Ansprüche, wobei die bevorzugte Verbindung X insbesondere aus denjenigen der folgenden durchschnittlichen Formel ausgewählt ist :
CH₂=CH(Me)₂SiO[Me₂SiO]_{x'}Si(Me)₂CH=CH₂
worin Me für eine Methylgruppe steht;
x' ≥ 0, x' vorzugsweise im Bereich von 0 bis 200 liegt, x' bevorzugt im Bereich von 10 bis 150 liegt.

8. Zusammensetzung nach einem der vorhergehenden Ansprüche, wobei das Organohydrogensiloxan die folgende durchschnittliche Formel aufweisen kann:
(R³₃SiO_{0,5})ₐ(R⁴₂SiO)_{b}(R⁴HSiO)_{c}
worin:
R³ für Wasserstoff oder R⁴ steht;
R⁴ für eine einwertige auf Kohlenwasserstoff basierende Gruppe mit 1 bis 10 Kohlenstoffatomen steht;
a ≥ 2;
b ≥ 0, vorzugsweise b = 1 bis 500, bevorzugt b = 1 bis 200; und
c ≥ 2, vorzugsweise c = 2 bis 200, bevorzugt c = 2 bis 100.

9. Zusammensetzung nach einem der vorhergehenden Ansprüche, wobei das Organohydrogensiloxan aus den Dimethylmethylhydrogenpolysiloxanen mit der folgenden durchschnittlichen Formel ansgewählt sein kann:
(CH₃)₃SiO[(CH₃)₂SiO]_{b}[(CH₃)HSiO]_{c}Si(CH₃)₃
worin b ≥ 0, vorzugsweise b = 1 bis 200, bevorzugt b = 1 bis 100; und
c ≥ 2, vorzugsweise c = 2 bis 100, bevorzugt c = 2 bis 50.

10. Zusammensetzung nach einem der vorhergehenden Ansprüche, wobei die Verbindungen X und Y in solchen Gehalten vorliegen, dass das Molverhältnis von SiH von Verbindung Y zu den ungesättigten Alkenylgruppen von Verbindung X größer als 1 ist, insbesondere im Bereich von 1 bis 10 liegt, vorzugsweise im Bereich von 1 bis 4 liegt und bevorzugt im Bereich von 2 bis 3 liegt.

11. Zusammensetzung nach einem der vorhergehenden Ansprüche, wobei es sich bei dem Hydrosilylierungskatalysator um ein Platingruppenmetall, das in einer Konzentration von 1 bis 500 Teilen pro Million, bezogen auf das Gesamtgewicht von Verbindung X, vorliegt, handelt.

12. Verbindung nach einem der vorhergehenden Ansprüche, **dadurch gekennzeichnet, dass** sie Cetrimoniumpropyltrimethoxysilanchlorid umfasst.

13. Zusammensetzung nach einem der vorhergehenden Ansprüche, **dadurch gekennzeichnet, dass** sie einen kosmetischen Bestandteil, der aus Ölen, Wachsen, pastösen Fettsubstanzen, Vitaminen, Verdickern, Gelierungsmitteln, Spurenelementen, Weichmachern, Sequestriermitteln, Duftstoffen, Alkalinisierungs- oder Ansäuerungsmitteln, Konservierungsmitteln, Tensiden, Antioxidantien, Fasern, Füllstoffen, Farbstoffen, filmbildenden Polymeren, kosmetischen Wirkstoffen wie bakteriziden Wirkstoffen oder Antiperspirantien, Neutralisationsmitteln, Emollientien, Feuchtigkeitsspendern und Mischungen davon ausgewählt ist, umfasst.

14. Kosmetisches Beschichtungsverfahren zur Pflege und/oder zum Schminken von Keratinmaterial(ien), umfassend zumindest das Auftragen einer Zusammensetzung nach einem der vorhergehenden Ansprüche auf das Keratinmaterial.

15. Verfahren nach dem vorhergehenden Anspruch, bei dem die Verbindungen X und Y miteinander reagieren, wenn sie mit dem zu behandelnden Keratinmaterial in Berührung stehen.

16. Verfahren nach Anspruch 14 oder 15, bei dem man die Zusammensetzung, die die Mikrokapseln umfasst, über einer durch vorheriges Aufbringen einer ersten kosmetischen Zusammensetzung auf die Keratinmaterialien gebildeten ersten Auftragung aufbringt.

## Revendications

1. Composition cosmétique comprenant, dans un milieu physiologiquement acceptable, au moins un composé X organopolysiloxane ayant au moins deux groupements insaturés alcényle, au moins un composé Y organohydrogénosiloxane et au moins un catalyseur d'hydrosilylation, et lesdits composés X et Y réagissant ensemble par une réaction d'hydrosilylation en présence du catalyseur, au moins un composé parmi les composés X et Y étant présent dans ladite composition sous une forme encapsulée dans des microcapsules à écorce de silice, ledit catalyseur étant associé audit composé X et/ou Y encapsulé ou étant encapsulé séparément, les microcapsules étant en suspension dans une phase aqueuse.

2. Composition selon la revendication 1, contenant au moins un composé X ou un composé Y sous une forme encapsulée.

3. Composition selon la revendication 1 ou 2, dans laquelle les composés X et Y sont tous deux présents sous des formes encapsulées séparées.

4. Composition selon l'une quelconque des revendications précédentes, dans laquelle une première portion des microcapsules comprend le composé X et le catalyseur et une deuxième portion comprend le composé Y, éventuellement associé au composé X.

5. Composition selon l'une quelconque des revendications précédentes, dans laquelle le composé X est choisi parmi les organopolysiloxanes comprenant au moins deux unités siloxane et ayant pour formule moyenne :
R²RₘSiO_{(4-m)/2}
dans laquelle
R est un groupe hydrocarboné ayant de 1 à 10 atomes de carbone,
R² est un groupe alcényle contenant de 2 à 12 atomes de carbone, et
m va de 0 à 2.

6. Composition selon l'une quelconque des revendications précédentes, dans laquelle le composé X est choisi parmi les composés suivantes :
CH₂=CH(Me)₂SiO[Me₂SiO]_{x'}Si(Me)₂CH=CH₂
CH₂=CH-(CH₂)₄-(Me)₂SiO[Me₂SiO]_{x'}Si(Me)₂-(CH₂)₄-CH=CH₂
Me₃SiO[(Me)₂SiO]_{x'}[CH₂=CH(Me)SiO]_{x"}SiMe₃
où Me est un groupe méthyle,
x' ≥ 0, de préférence x' va de 0 à 200, préférentiellement x' va de 10 à 150, et
x" ≥ 2, de préférence x" va de 2 à 50, préférentiellement x " va de 2 à 10.

7. Composition selon l'une quelconque des revendications précédentes, dans laquelle le composé X préféré est en particulier choisi parmi ceux de formule moyenne suivante :
CH₂=CH(Me)₂SiO[Me₂SiO]_{x'}Si(Me)₂CH=CH₂
dans laquelle Me est un groupe méthyle,
x' ≥ 0, de préférence x' va de 0 à 200, préférentiellement x' va de 10 à 150.

8. Composition selon l'une quelconque des revendications précédentes, dans laquelle l'organohydrogénosiloxane peut avoir la formule moyenne suivante :
(R³₃SiO_{0.5})ₐ(R⁴₂SiO)_{b}(R⁴HSiO)_{c}
dans laquelle
R³ est hydrogène ou R⁴,
R⁴ est un groupe hydrocarboné monovalent, ayant de 1 à 10 atomes de carbone,
a ≥ 2,
b ≥0, de préférence b = 1 à 500, préférentiellement b = 1 à 200, et
c ≥ 2, de préférence c = 2 à 200, préférentiellement c = 2 à 100.

9. Composition selon l'une quelconque des revendications précédentes, dans laquelle l'organohydrogénosiloxane peut être choisi parmi les diméthyl méthyl-hydrogène polysiloxanes ayant pour formule moyenne :
(CH₃)₃SiO[(CH₃)₂SiO]_{b}[(CH₃)HSiO]_{c}Si(CH₃)₃
dans laquelle b ≥ 0, de préférence b = 1 à 200, préférentiellement b = 1 à 100,
et c ≥ 2, de préférence c = 2 à 100, préférentiellement c = 2 à 50.

10. Composition selon l'une quelconque des revendications précédentes, dans laquelle les composés X et Y sont présents en des teneurs telles que le ratio molaire de SiH du composé Y par rapport aux groupes alcényle insaturés du composé X est supérieur à 1, notamment allant de 1 à 10, de préférence allant de 1 à 4, et préférentiellement allant de 2 à 3.

11. Composition selon l'une quelconque des revendications précédentes, dans laquelle le catalyseur d'hydrosilylation est un métal du groupe de platine présent en une concentration de 1 à 500 parties par million, par rapport au poids total du composé X.

12. Composition selon l'une quelconque des revendications précédentes, **caractérisée par le fait qu'**elle comprend du chlorure de cétrimoniumpropyltriméthoxysilane.

13. Composition selon l'une quelconque des revendications précédentes, **caractérisée par le fait qu'**elle comprend un ingrédient cosmétique choisi parmi les huiles, les cires, les corps gras pâteux, les vitamines, les épaississants, les gélifiants, les oligo-éléments, les adoucissants, les séquestrants, les parfums, les agents alcalinisants ou acidifiants, les conservateurs, les tensioactifs, les anti-oxydants, les fibres, les charges, les matières colorantes, les polymères filmogènes, les actifs cosmétiques comme les actifs bactéricides ou anti-transpirants, les neutralisants, les émollients, les hydratants, et leurs mélanges.

14. Procédé cosmétique de revêtement pour le soin et/ou le maquillage de matière (s) kératinique(s) comprenant au moins l'application sur ladite matière kératinique d'une composition selon l'une quelconque des revendications précédentes.

15. Procédé selon la revendication précédente, dans lequel les composés X et Y réagissent ensemble lorsqu'ils sont en contact avec la matière kératinique à traiter.

16. Procédé selon l'une des revendications 14 ou 15, dans lequel la composition comprenant les microcapsules est appliquée par-dessus un premier dépôt formé par application préalable sur les matières kératiniques d'une première composition cosmétique.
